# EUROPEAN PATENT SPECIFICATION

(11) **EP 1 468 092 B1**
(45) Date of publication and mention of the grant of the patent: **11.01.2012**
(21) Application number: 03701530.2
(22) Date of filing: 20.01.2003
(51) Int. Cl.: C12N 15/54, C12N 9/12, A61K 38/43, G01N 33/68, C12N 1/21, C12Q 1/68, A61P 3/10

(54) **PIM-3 KINASE AS A TARGET FOR TYPE 2 DIABETES MELLITUS**
PIM-3 KINASE ALS ZIEL FÜR TYPE 2 DIABETES MELLITUS
PIM-3 KINASE EN TANT QUE CIBLE POUR LE DIABETE DE TYPE 2

(30) Priority: 19.01.2002 EP 02001401
(43) Date of publication of application: 20.10.2004
(73) Proprietor: Sanofi-Aventis Deutschland GmbH, 65929 Frankfurt am Main (DE)
(72) Inventor: KORN, Marcus, 63065 Offenbach (DE); MUELLER, Guenter, 65843 Sulzbach (DE); SCHNEIDER, Rudolf, 65527 Niedernhausen (DE); TSCHANK, Georg, 55270 Essenheim (DE)
(86) International application number: PCT/EP2003/000492
(87) International publication number: WO 2003/060130

(56) References cited:
- EP-A- 0 911 391
- WO-A-02/093173
- US-A- 6 143 540
- DATABASE EMBL [Online] 5 December 2001 (2001-12-05) R. STRAUSBERG: "Mus musculus, serine threonine kinase pmi3, clone MGC:27707" retrieved from EBI,HINXTON, UK Database accession no. BC017621 XP002203189
- DATABASE EMBL [Online] 1 August 1998 (1998-08-01) U. KONIETZKO ET AL: "Serine Threonine Kinase (Protein kinase KID-1)" retrieved from EBI, HINXTON, UK Database accession no. 070444 XP002203190
- NADLER SAMUEL T ET AL: "The expression of adipogenic genes is decreased in obesity and diabetes mellitus." PROCEEDINGS OF THE NATIONAL ACADEMY OF SCIENCES OF THE UNITED STATES, vol. 97, no. 21, 10 October 2000 (2000-10-10), pages 11371-11376, XP002203186 October 10, 2000 ISSN: 0027-8424
- KONIETZKO UWE ET AL: "Pim kinase expression is induced by LTP stimulation and required for the consolidation of enduring LTP." EMBO (EUROPEAN MOLECULAR BIOLOGY ORGANIZATION) JOURNAL, vol. 18, no. 12, 15 June 1999 (1999-06-15), pages 3359-3369, XP002203187 ISSN: 0261-4189 cited in the application
- FELDMAN JONATHAN D ET AL: "KID-1, a protein kinase induced by depolarization in brain." JOURNAL OF BIOLOGICAL CHEMISTRY, vol. 273, no. 26, 26 June 1998 (1998-06-26), pages 16535-16543, XP002203188 ISSN: 0021-9258 cited in the application
- DATABASE EMBL [Online] 27 June 2002 (2002-06-27) "protein sequence related to human protein kinase gene KID-1" retrieved from HINXTON, US Database accession no. AA020524 XP002248727 & CN 1 328 134 A ((UYFU-N) UNIV FUDAN) 26 December 2001 (2001-12-26)
- DATABASE EMBL [Online] 9 April 2002 (2002-04-09) R. STRAUSBERG: "Mus musculus, clone MGC:375217 IMAGE:4985551, mRNA, complete cds" retrieved from EBI, HINXTON, UK Database accession no. BC026639 XP002248728

## Description

The present invention relates to a novel use of PIM-3 kinase.

The rat PIM-3 (originally termed KID-1, KID "kinase induced by depolarisation"), frog PIM-1, and human and murine PIM-1 are all known to have serine/threonine protein kinase activity in in vitro phosphorylation assays. The high polypeptide sequence similarity between human, murine and rat PIM-3, frog PIM-1, and human and murine PIM-1, demonstrates that human and murine PIM-3 are a serine/threonine protein kinase.

Rat PIM-3 is described by Feldman, J. D. et al. (J. Biol. Chem. (1998) 273, 16535 - 16543). Rat PIM-3 is induced in specific regions of the hippocampus and cortex in response to kainic acid and electroconvulsive shock suggesting that PIM-3 is involved in neuronal function, synaptic plasticity, learning, and memory as well as kainic acid seizures and some nervous system-related diseases such as seizures and epilepsy.

US 6,143,540, Konietzko, U. et al. (EMBO (1999) 18, 3359-3369 Database EMBM accession no. 8C017621 and Eichmann, A. (Oncogene (2000) 19, 1215-1224) also refer to PIM-3 kinase. WO 02/093173 refers to PIM-3 kinase for screening for pain regulating substances.

The present invention provides novel uses of PIM-3, as specified in the claims.

The present invention discloses human and murine PIM-3 sequence. SEQ ID NO. 1 depicts the DNA sequence and SEQ ID NO. 2 the predicted amino acid sequence of human PIM-3. The open reading frame of SEQ ID NO:1 extends from nucleotide 17 to nucleotide 995 (SEQ ID NO. 3).

SEQ ID NO. 5 depicts the DNA sequence and SEQ ID NO. 6 the predicted amino acid sequence of murine PIM-3. The open reading frame of SEQ ID NO. 5 extends from nucleotide 199 to nucleotide 1177 (SEQ ID NO. 7).

The present invention demonstrates that expression of the rat PIM-3 gene is decreased in adipocytes in two independent models of insulin resistance. Treatment with an insulin sensitizer causes an increase in PIM-3 gene expression in murine 3T3-L1 cells. Because human and murine PIM-3 is the species ortholog of rat PIM-3 human and murine PIM-3 is involved in some or all of the processes and diseases in which rat PIM-3 is involved. PIM-3, in particular human and murine PIM-3 is involved in development of insulin resistance. In addition PIM-3, in particular human and murine PIM-3 is involved in development of type 2 diabetes mellitus. In addition, the human and murine PIM-3 paralogs, the PIM-1 proteins, are proto-oncogenes. Consequently, PIM-3. in particluar human and murine PIM-3 are involved in cell growth regulation, cancer, and related pathways and diseases.

The present invention relates to the use of PIM-3 encoding nucleic acid molecules, and PIM-3 proteins in
a) screening assays for identifying compounds that modulate insulin resistance or type 2 diabetes mellitus;
b) detection assays for detecting insulin resistance or type 2 diabetes mellitus (e.g. chromosomal mapping, tissue typing, forensic biology); c) predictive medicine (prediction of insulin resistance or type 2 diabetes mellitus by e.g. diagnostic assays, prognostic assays, monitoring clinical trials, and pharmacogenomics).

Specificalles, the present invention relates to the use of PIM-3 as a screening agent for identifying anti-diabetes mellitus drugs, e.g. the use of PIM-3 encoding DNA which comprises nucleotide sequence SEQ ID NO. 1,5 or 9 or a polypeptide having PIM-3 activity and having amino acid sequence SEQ ID 10 NO.2, 6 or 10 for such purpose.

The present invention further relates to the PIM-3 polypeptide for use in the treatment of insulin resistance or type 2 diabetes mellitus wherein the polypeptide has amino acid sequence SEQ. ID. NO. 2, 6 or 10.

The present invention further discloses an isolated nucleic acid molecule comprising a nucleotide sequence selected from
a) SEQ ID NO. 5 and
b) SEQ ID NO.7.

The present invention furthermore discloses vectors and host cells comprising the respective DNA sequences or parts thereof.

The present invention further discloses polypeptides having PIM-3 activity, said polypeptides being selected from
a) a polypeptide having amino acid sequence SEQ ID NO. 6,
b) a fusion polypeptide comprising an amino acid sequence comprising the amino acids of sequence SEQ ID NO. 6.

The present invention further discloses the use of PIM-S for predicting insulin resistance or type 2 diabetes mellitus.

PIM-3 protein which interacts with other cellular proteins can thus be used as a target for developing therapeutic molecules for modulating PIM-3 protein in cells expressing PIM-3 protein or cells involved in the PIM-3 pathway, e.g., adipocytes. Nucleic acid molecules of the invention can be used to express PIM-3 protein (e.g. via a recombinant expression vector in a host.cell in gene therapy applications), to detect PIM-3 mRNA (e.g. in a biological sample) or a genetic lesion in an PIM-3 gene, and to modulate PIM-3 activity.

PIM-3 proteins can be used to screen drugs or compounds which modulate the PIM-3 activity or expression as well as to treat disorders characterized by insufficient or excessive production of PIM-3 protein or production of PIM-3 protein forms which have decreased or aberrant activity compared to PIM-3 wild type protein.

The invention provides methods (also referred to herein as a "screening assay") for identifying modulators, i.e. candidate or test compounds or agents (e.g. peptides, peptidomimetics, small molecules or other drugs) which bind to PIM-3 proteins or have a stimulatory or inhibitory effect on, for example, PIM-3 expression or PIM-3-activity.

In one embodiment, the invention provides assays for screening candidate or test compounds which bind to or modulate the activity of a PIM-3 protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection.

In an embodiment, an assay of the present invention is a cell-free assay comprising contacting a PIM-3 protein or biologically active portion thereof with a test compound and determining the ability of the test compound to bind to the PIM-3 protein or biologically active portion thereof. Binding of the test compound to the PIM-3 protein can be determined either directly or indirectly.

In an other embodiment, the assay includes contacting the PIM-3 protein or biologically active portion thereof with a known compound which binds PIM-3 to form an assay mixture, contacting the assay mixture with a test compound, and determining the ability of the test compound to interact with a PIM-3 protein, wherein determining the ability of the test compound to interact with a PIM-3 protein comprises determining the ability of the test compound to preferentially bind to PIM-3 or biologically active portion thereof as compared to the known compound.

In another embodiment, an assay is a cell-free assay comprising contacting PIM-3 protein or biologically active portion thereof with a test compound and determining the ability of the test compound to modulate (e.g., stimulate or inhibit) the activity of the PIM-3 protein or biologically active portion thereof. Determining the ability of the test compound to modulate the activity of PIM-3 can be accomplished, for example, by determining the ability of the PIM-3 protein to bind to a PIM-3 target molecule, this means determining direct binding. In an alternative embodiment, determining the ability of the test compound to modulate the activity of PIM-3 can be accomplished by determining the ability of the PIM-3 protein to further modulate an PIM-3 target molecule. For example, the catalytic/enzymatic activity of the target molecule on an appropriate substrate can be determined.

In another embodiment, the cell-free assay comprises contacting the PIM-3 protein or biologically active portion thereof with a known compound which binds PIM-3 to form an assay mixture, contacting the assay mixture with a test compound, and determining the ability of the test compound to interact with the PIM-3 protein, wherein determining the ability of the test compound to interact with the PIM-3 protein comprises determining the ability of the PIM-3 protein to preferentially bind to or modulate the activity of a PIM-3 target molecule.

Phosphoaminoacid analysis of the phosphorylated substrate can also be performed in order to determine which residues on the PIM-3 substrate are phosphorylated. Briefly, the radiophosphorylated protein band can be excised from the SDS gel and subjected to partial acid hydrolysis. The products can then be separated by one-dimensional electrophoresis and analyzed on, for example, a phosphoimager and compared to ninhydrin-stained phosphoaminoacid standards.

In another embodiment of the invention, the cell free assay determines the ability of the PIM-3 protein to phosphorylate an PIM-3 target molecule by, for example, an in vitro kinase assay. Briefly, a PIM-3 target molecule, e.g., an immunoprecipitated PIM-3 target molecule from a cell line expressing such a molecule, can be incubated with the PIM-3 protein and radioactive ATP.

In another embodiment, an assay is a cell-based assay in which a cell which expresses a soluble form of PIM-3 protein, or a biologically active portion thereof, is contacted with a test compound and the ability of the test compound to bind to a PIM-3 protein determined. The cell, for example, can be a yeast cell or a cell of mammalian origin. Determining the ability of the test compound to bind to the PIM-3 protein can be accomplished, for example, by coupling the test compound with a radioisotope or enzymatic label such that binding of the test compound to the PIM-3 protein or biologically active portion thereof can be determined by detecting the labeled compound in a complex, e. g. with .sup. 125 I, .sup. 35 S, .sup. 14 C, or .sup. 3 H, or enzymatically with, for example, horseradish peroxidase, alkaline phosphatase, or luciferase.

In another embodiment, an assay is a cell-based assay comprising contacting a cell expressing a soluble form of PIM-3 protein, or a biologically active portion thereof, with a test compound and determining the ability of the test compound to modulate (e.g., stimulate or inhibit) the activity of the PIM-3 protein or biologically active portion thereof. Determining the ability of the test compound to modulate the activity of PIM-3 or a biologically active portion thereof can be accomplished, for example, by determining the ability of the PIM-3 protein to bind to or interact with an PIM-3 target molecule.

As used herein, a "target molecule" is a molecule with which an PIM-3 protein binds or interacts in nature, for example, a substrate molecule phosphorylated by PIM-3 protein in the interior of a cell which expresses a PIM-3 protein, the intracellular domains of transmembrane receptors, a molecule associated with the internal surface of a cell membrane or a cytoplasmic molecule. A PIM-3 target molecule can be a non-PIM-3 molecule or a PIM-3 protein or polypeptide of the present invention. In one embodiment, a PIM-3 target molecule is a component of a signal transduction pathway which mediates transduction of a signal.

In an embodiment, determining the ability of the PIM-3 protein to bind to or interact with a PIM-3 target molecule can be accomplished by determining the activity of the target molecule. For example, the activity of the target molecule can be determined by detecting induction of a cellular second messenger of the target (e.g., intracellular Ca.sup.2+, diacylglycerol, IP3, etc.), detecting catalytic/enzymatic activity of the target on an appropriate substrate, detecting the induction of a reporter gene (e.g., a PIM-3-responsive regulatory element operably linked to a nucleic acid encoding a detectable marker, e.g. luciferase), or detecting a cellular response, for example, cellular differentiation, or cell proliferation.

In various formats of the assay methods of the present invention, it may be desirable to immobilize either PIM-3 or its target molecule to facilitate separation of complexed from uncomplexed forms of one or both of the proteins, as well as to accommodate automation of the assay. Binding of a test compound to PIM-3, or interaction of PIM-3 with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/PIM-3 fusion proteins or glutathione-S-transferase/target fusion proteins can be adsorbed onto glutathione sepharose beads or glutathione derivatized microtitre plates, which are then combined with the test compound or the test compound and either the non-adsorbed target protein or PIM-3 protein, and the mixture incubated under conditions conducive to complex formation (e.g., at physiological conditions for salt and pH). Following incubation, the beads or microtitre plate wells are washed to remove any unbound components and complex formation is measured either directly or indirectly. Alternatively, the complexes can be dissociated from the matrix, and the level of PIM-3 binding or activity determined using standard techniques.

Other techniques for immobilizing proteins on matrices can also be used in the screening assays of the invention. For example, either PIM-3 or its target molecule can be immobilized utilizing conjugation of biotin and streptavidin. Biotinylated PIM-3 or target molecules can be prepared from biotin-NHS (N-hydroxy-succinimide) using techniques well known in the art. Alternatively, antibodies reactive with PIM-3 or target molecules but which do not interfere with binding of the PIM-3 protein to its target molecule can be derivatized to the wells of the plate, and unbound target or PIM-3 trapped in the wells by antibody conjugation. Methods for detecting such complexes, in addition to those described above for the GST-immobilized complexes, include immunodetection of complexes using antibodies reactive with the PIM-3 or target molecule, as well as enzyme-linked assays which rely on detecting an enzymatic activity associated with the PIM-3 or target molecule.

In another embodiment, modulators of PIM-3 expression are identified in a method in which a cell is contacted with a candidate compound and the expression of PIM-3 mRNA or protein in the cell is determined. The level of expression of PIM-3 mRNA or protein in the presence of the candidate compound is compared to the level of expression of PIM-3 mRNA or protein in the absence of the candidate compound. The candidate compound can then be identified as a modulator of PIM-3 expression based on this comparison. For example, when expression of PiM-3 mRNA or protein is greater (statistically significantly greater) in the presence of the candidate compound than in its absence, the candidate compound is identified as a stimulator of PIM-3 mRNA or protein expression. Alternatively, when expression of PIM-3 mRNA or protein is less (statistically significantly less) in the presence of the candidate compound than in its absence, the candidate compound is identified as an inhibitor of PIM-3 mRNA or protein expression. The level of PIM-3 mRNA or protein expression in the cells can be determined by methods described herein for detecting PIM-3 mRNA or protein.

In another aspect of the invention, the PIM-3 proteins or polypeptides thereof can be used as "balt proteins" in a two-hybrid assay or three hybrid assay, to identify other proteins, which bind to or interact with PIM-3 ("PIM-3-binding proteins" or "PIM-3-bp") and modulate PIM-3 activity. Such PIM-3-binding proteins are also likely to be involved in the propagation of signals by the PIM-3 proteins as, for example, upstream or downstream elements of the PIM-3 pathway. The invention also provides for the use of proteins that interact with PIM-3, e.g., two-hybrid interactors with PIM-3, as baits in two-hybrid screens and the identification of PIM-3 interacting protein interacting proteins. PIM-3 interacting protein interacting proteins are likely to be involved in the PIM-3 signal transduction pathway.

The present description also relates to the field of predictive medicine in which diagnostic assays, prognostic assays, pharmacogenomics, and monitoring clinical trials are used for prognostic (predictive) purposes to thereby treat an individual prophylactically. Accordingly, one aspect of the present invention relates to diagnostic assays for determining PIM-3 protein and/or nucleic acid expression as well as PIM-3 activity, in the context of a biological sample (e.g., blood, serum, cells, tissue form the individual, preferably human) to thereby determine whether an individual is afflicted with a disease or disorder, or is at risk of developing a disorder, associated with aberrant PIM-3 expression or activity. The invention also provides for prognostic (or predictive) assays for determining whether an individual is at risk of developing a disorder associated with PIM-3 protein, nucleic acid expression or activity. For example, mutations in a PIM-3 gene can be assayed in a biological sample. Such assays can be used for prognostic or predicative purpose to thereby prophylactically treat an individual prior to the onset of a disorder characterized by or associated with PIM-3 protein, nucleic acid expression or activity.

The present description also relates to methods for determining PIM-3 protein, nucleic acid expression or PIM-3 activity in an individuals biological sample to thereby select appropriate therapeutic or prophylactic agents for that individual (referred to herein as "pharmacogenomics"). Pharmacogenomics allows for the selection of agents (e.g., drugs) for therapeutic or prophylactic treatment of an individual based on the genotype of the individual (e.g., the genotype of the individual examined to determine the ability of the individual to respond to a particular agent.)

The present description relates to monitoring the influence of agents (e.g., drugs or other compounds) on the expression or activity of PIM-3 in clinical trials.

An exemplary method for detecting the presence or absence of PIM-3 in a biological sample involves obtaining a biological sample from a test subject and contacting the biological sample with a compound or an agent capable of detecting PIM-3 protein or nucleic acid (e.g., mRNA, genomic DNA) that encodes PIM-3 protein such that the presence of PIM-3 is detected in the biological sample. An agent for detecting PIM-3 mRNA or genomic DNA can be a labeled nucleic acid probe capable of hybridizing to PIM-3 mRNA or genomic DNA.

An agent for detecting PIM-3 protein can be an antibody capable of binding to PIM-3 protein, preferably an antibody with a detectable label. Antibodies can be polyclonal, or more preferably, monoclonal. An Intact antibody, or a fragment thereof (e.g., Fab or F(ab').sub.2) can be used.

The term "biologicial sample" is Intended to include tissues, cells and biological fluids isolated from an individual, as well as tissues, cells and fluids present within an individual. That is, the detection method of the invention can be used to detect PIM-3 mRNA, protein, or genomic DNA in a biological sample e.g. in vitro as well as or in vivo. In one embodiment, the biological sample contains protein molecules from the test subject. Alternatively, the biological sample can contain mRNA molecules from the test subject or genomic DNA molecules from the test subject. A biological sample is e.g. a biopsy from adipose tissue isolated by conventional means from a subject.

In another embodiment, the methods further involve obtaining a control biological sample from a control subject, contacting the control sample with a compound or agent capable of detecting PIM-3 protein, mRNA, or genomic DNA, such that the presence of PIM-3 protein, mRNA or genomic DNA is detected in the biological sample, and comparing the presence of PIM-3 protein, mRNA or genomic DNA in the control sample with the presence of PIM-3 protein, mRNA or genomic DNA in the test sample.

The specification also encompasses kits for detecting the presence of PIM-3 in a biological sample (a test sample). Such kits can be used to determine if an individual is suffering from or is at increased risk of developing a disorder associated with insulin resistance or type 2 diabetes. For example, the kit can comprise a labeled compound or agent capable of detecting PIM-3 protein or mRNA in a biological sample and means for determining the amount of PIM-3 in the sample (e.g., an anti-PIM-3 antibody or an oligonucleotide probe which binds to DNA encoding PIM-3, e.g., SEQ ID NO:1, SEQ ID NO:3, SEQ ID NO. 5 or SEQ ID NO. 7).

The methods described herein can furthermore be utilized as diagnostic or prognostic assays to identify subjects having or at risk of developing a disease or disorder associated with aberrant PIM-3 expression or activity. For example, the assays described herein, such as the preceding diagnostic assays or the following assays, can be utilized to identify a subject having or at risk of developing insulin resistance or type 2 diabetes. Thus, the present invention provides a method in which PIM-3 protein or nucleic acid (e.g., mRNA, genomic DNA) is detected in a test sample from an individual, wherein the presence of PIM-3 protein or nucleic acid is diagnostic for the individual for having or at risk of developing a disease or disorder associated with aberrant PIM-3 expression or activity. As used herein, a "test sample" refers to a biological sample obtained from a subject of interest. For example, a test sample can be a biological fluid (e.g., serum), cell sample, or tissue.

Furthermore, the prognostic assays can be used to determine whether a subject can be administered an agent (e.g., an agonist, antagonist, peptidomimetic, protein, peptide, nucleic acid, small molecule, or other drug candidate) to treat insulin resistance or type 2 diabetes. For example, such methods can be used to determine whether a subject can be effectively treated with a specific agent or class of agents (e.g., agents of a type which decrease PIM-3 activity).

Agents, or modulators which have a stimulatory or inhibitory effect on PIM-3 activity (e.g., PIM-3 gene expression) as identified by a screening assay can be used for preparing a pharmaceutical which is useful for treating (prophylactically or therapeutically) disorders (e.g., disorders involving cells or tissues in which PIM-3 is expressed, such as adipocytes) associated with aberrant PIM-3 activity. In conjunction with such treatment, the pharmacogenomics (i.e. the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of PIM-3 protein, expression of PIM-3 nucleic acid, or mutation content of PIM-3 genes in an individual can be determined to thereby select appropriate agent(s) for therapeutic or prophylactic treatment of the individual.

Monitoring the influence of agents (e.g., drugs, compounds) on the expression or activity of PIM-3 (e.g., the ability to modulate aberrant cell proliferation and/or differentiation) can be applied not only in basic drug screening, but also in clinical trials.

An anti type 2 diabetes agent that modulates PIM-3 protein activity can be an agent, such as a small molecule, e.g., a small molecule that modulates the protein kinase activity of PIM-3, a nucleic acid or a protein, a naturally-occurring cognate ligand of a PIM-3 protein, a peptide, or a PIM-3 peptidomimetic. In one embodiment, the agent stimulates one or more of the biological activities of PIM-3 protein. Examples of such stimulatory agents include small molecules that stimulate one or more activities of PIM-3, e.g., the PIM-3 protein kinase activity, active PIM-3 protein and a nucleic acid molecule encoding PIM-3 that has been introduced into the cell. In another embodiment, the agent inhibits one or more of the biological activities of PIM-3 protein. Examples of such inhibitory agents include a small molecule that inhibits one or more PIM-3 activities e.g., PIM-3 protein kinase activity, antisense PIM-3 nucleic acid molecules and anti-PIM-3 antibodies.

This invention is further illustrated by the following examples which should not be construed as limiting.

### Examples

### Example 1

### Determination of the Nucleotide Sequence of human and murine PIM-3

Rat PIM-3 nucleotide sequence (AF057026, NM_022602, SEQUENCE ID NO:9) was used to query a proprietary database using the BLASTN program with the BLOSUM62 matrix. This proprietary database is based on a proprietary cDNA library which is constructed in standard cloning vectors. The most closely related cDNA clones identified by this BLASTN were sequenced. The cDNA sequences were assembled into a contig. The human PIM-3 sequence was determined from the consensus sequence of this contig, further analysis of this contig revealed a cDNA sequence 1977 bp in length. That human PIM-3 cDNA contains a 978 base pair open reading frame predicted to encode a novel 326 amino acid protein.

Rat PIM-3 nucleotide sequence was also used to query the public UNIGENE Mouse database using the BLASTN program with the BLOSUM62 matrix. Some closely related EST sequences were identified by this BLASTN. Sequence information of this ESTs was used to screen a mouse embryo cDNA library constructed using the Gene Trapper II Technology (Life Technologies, Karlsruhe, Germany). A cDNA clone was sequenced, and the cDNA sequences were assembled into a contig. The murine PIM-3 sequence was determined from the consensus sequence of this contig, further analysis of this contig and exclusion of an intron sequence revealed a cDNA sequence 2236 bp in length. That murine PIM-3 cDNA contains a 978 base pair open reading frame predicted to encode a novel 326 amino acid protein.

### Example 2

### Characterization of the human and the murine PIM-3 protein

In this example, the predicted amino acid sequence of human PIM-3 and the murine PIM-3 protein was compared to amino acid sequences of known motifs and/or domains present in proteins and to the polypeptide sequences of known proteins. Polypeptide domains and/or motifs present in human PIM-3 and murine PIM-3 were identified as were proteins with significant amino acid similarities to human PIM-3 and murine PIM-3. In addition, the molecular weight of the human PIM-3 and the murine PIM-3 protein was predicted.

The human and the murine nucleotide sequences (SEQ ID NO.1; SEQ ID NO.5), identified as described above, encode a 326 amino acid protein (SEQ ID NO.2 and SEQ ID NO.6). Human and murine PIM-3 has a predicted MW of about 35.9 kDa, respectively, not including post-translational modifications. To check for evidences for a putative kinase activity and for possible posttranslational modification sites, the human and the murine polypeptide sequences of SEQ ID NO.2 and SEQ ID NO.6, respectively, were analyzed using the PROSITE database of protein patterns, as well as using IMPALA and PFAM.

Searching the PROSITE database revealed the presence of one cAMP and cGMP dependent protein kinase phosphorylation site from amino acids 260-263 of SEQ ID NO.2 and SEQ ID NO.6; three Casein kinase II phosphorylation sites from amino acids 202-205, 211-214 and 321-324 of SEQ ID NO.2 and four Casein kinase II phosphorylation sites from amino acids 202-205, 211-214 299-302 and 321-324 of SEQ ID NO.6; ten N-myristoylation sites from amino acids 43-48, 49-54, 52-57, 57-62, 63-68, 80-85, 98-103, 101-106, 295-300 and 316-321 of SEQ ID NO.2 and SEQ ID NO.6; three Protein kinase C phosphorylation sites from amino acids 137-139, 275-277 and 279-281 of SEQ ID NO.2 and SEQ ID NO.6; one tyrosine kinase phosphorylation site from amino acids 33-40 of SEQ ID NO.2 and SEQ ID NO.6; one protein kinases signature and profile (ATP binding site) from amino acids 46-69 of SEQ ID NO.2 and SEQ ID NO.6; one serine/threonine protein kinase active site signature from amino acids 166-178 of SEQ ID NO.2 and SEQ ID NO.6. The search using IMPALA revealed the presence of one eukaryotic protein
kinase domain from amino acid 40-293 (SEQ ID NO.4 and SEQ ID NO.8, respectively), of SEQ ID NO.2 and SEQ ID NO.6; with a score of 186 bits and Expect value of 8e-49 and with a score of 184 bits and Expect value of 4e-48, respectively. The search using PFAM revealed also the presence of one eukaryotic protein kinase domain from amino acid 40-293, of SEQ ID NO.2 and SEQ ID NO.6; with a score of 262,5 and E-value of 5.7e-75 and with a score of 261,1 bits and E-value of 1.5e-74, respectively.

The human, murine and rat PIM-3 polypeptide sequences (SEQ ID NO.2, SEQ ID NO.6 and SEQ ID NO.10) were aligned in a pair wise Clustal W alignment analysis using blosum as the protein weight matrix. Thereby, human PIM-3 was found to be 95 % identical to murine and rat PIM-3 (AF057026, NM_022602, SEQ ID NO.10)with a score of 2011, murine PIM-3 was found to be 99 % identical to rat PIM-3 (AF057026, NM_022602, SEQ ID NO.10)with a score of 2074. The human and the murine PIM-3 polypeptide sequences of SEQ ID NO.2 and of SEQ ID NO.6 was also used to query the Swissprot database of protein sequences using the BLASTP program with the BLOSUM62 matrix. The four most closely related proteins to human and murine PIM-3 identified by this BLASTP analysis are listed: Human and murine PIM-3 was found to be 76% identical to Xenopus laevis (frog) PIM-1 (Q91822; SEQ ID NO.11) with a score of 518, 72% identical to rat PIM-1 (P26794; SEQ ID NO.12) with a score of 442, 71% identical to human PIM-1 (P11309; SEQ ID NO.13) with a score of 441 and 71% identical to murine PIM-1 (P06803; SEQ ID NO.14) with a score of 436 and 438, respectively.

### Example 3:

### Gene Expression of PIM-3 in an in vivo model of insulin resistance and type 2 diabetes mellitus

Zucker diabetic fatty (ZDF) rats are well known animal model carrying a homozygous defect in the leptin receptor fa gene. This rat strain develops age dependent an insulin resistant / hyperinsulinaemic state which than progresses to overt type 2 diabetes mellitus / hyperglycaemic state. To identify genes the expression of which is either induced or repressed and so may contribute or mark the development of insulin resistance or type 2 diabetes mellitus, gene expression profiles of ZDF rats and their lean heterozygous control littermates were collected using oligonucleotide array based profiling technique.

### Material and methods:

Zucker Diabetic Fatty (ZDF/Gmi.TM.-fa/fa) male rats as well as their lean male fa+/fa-counterparts, used as healthy controls, aged 6,8 and 13 weeks were obtained from Genetic Models Inc. (Indianapolis, IND., US).

Before the animals were included into the study they were kept under standard animal house conditions for one week. For collection of epidydimal fat tissue and blood samples the animals were killed by cervical dislocation. 6 ZDF rats and 6 lean fa+/fa-littermates per age group were used for gene expression analysis as described below.

### Tissue collection and RNA isolation:

Following cervical dislocation, epididymal fat pads were surgically removed, portioned and quickly transferred in suitable tubes containing sufficient volumes of RNA later (Ambion, TX, US). Samples from each animal were stored individually. Long term storage of the samples was performed at minus 80.degree.C.

Total cellular RNA was extracted from adipose tissue using Rneasy Mini kit (Qiagen, Hilden, Germany) according to the manufacturers recommendations for RNA isolation from fat tissue. RNA was eluted twice in 50.mul RNAse free water, RNA concentration was determined spectroscopically (A.sub.260).

For further purification, RNA solution was filled up to 100 µl total volume with RNase free water. RNA clean up was performed according to the manufacturers instructions using Qiagen Rneasy Mini kit. RNA was eluted twice in 50 µl RNase free water, RNA concentration was determined spectroscopically (A.sub.260).

For concentration of the RNA eluate, NH.sub.4 acetate and ethanol were added, and RNA was precipitated overnight in an ethanol-dry ice bath. RNA was collected by centrifugation at maximum speed at 4.degree.C. Pelleted RNA was washed twice with 80 % ethanol, air dried and dissolved in a small volume of RNAse free water. Rnase concentration was determined spectroscopically (A.sub.260).

### Gene expression profiling:

The general use of oligonucleotides arrays for gene expression monitoring has been described in US 6,177,248. In our practical application, the used microarrays contain desoxynucleotide sequences that represent approximately 8000 known genes or EST clusters. Each gene or EST sequence is represented by up to 20 pairs of oligonucleotides, each pair consisting of one oligo that matches to a segment of the transcript, and a control oligo that contains a centrally located 1 bp mismatch. For rat, 3 arrays (RG U34A, RG U34B and RG U34C) representing approximately 24000 gene and EST sequences in total, derived from a database of known genes or EST sequences are provided by Affymetrix, Santa Clara, CA, US.

### cRNA preparation for hybridization:

RNA was obtained from epididymal fat as decribed above. An oligo dT primer containing a T7 promotor site was added to total cellular RNA (10.mu.g)+-. After annealing of the primer, the RNA was subsequently reverse transcribed using Superscript choice reverse transcriptase, following the manufacturers instructions.

After extraction with phenol:chloroform:isoamylalcohol, using phase lock gel tubes (Eppendorf, Hamburg, Germany), and ethanol precipitation, the cDNA was collected by centrifugation and washed twice with 80 % ethanol. The pellet was dissolved in Rnase free water and transcribed into biotinylated cRNA using the Enzo High Yield labelling transcription kit (Enzo Diagnostics, Farmingdale NY, US) or MEGAscript T7 high yield transcription kit (Ambion, Austin, TX, US) according the manufacturers
instructions. For the latter application, biotin labelled UTP and CTP (Sigma, Munich, Germany) plus unlabelled ATP and GTP was used in a molar ratio of 1:3 (labelled vs unlabelled). cRNA was then precipitated and washed as described above. Finally, the precipitated, air dried cRNA was dissolved in a small volume of RNAse free water. RNA concentration was determined spectroscopically (A.sub260), and size distribution was checked by agarose gelelectrophoresis. Subsequently, cRNA was hydrolwyzed to an average size of 50 nucleotides in length by incubating for 25 minutes in 40 mM Trisacetate pH 8.1, 100 mM potassium acetate, 30 mM magnesium acetate at 94.degree.C. 20 µg of the fragmented cRNA was used to set up of a hybridisation cocktail according to the instructions of Affymetrix. Prior hybridization, RNA samples were heated in the hybridization cocktails to 99.degree.C for 10 min, placed on ice for 5 min, and allowed to equilibrate to room temperature before being placed in the hybridization flow cell. The hybridsation cocktail was then hybridised to a microarray at 45°C and 60 rpm in a hybridisation oven overnight. After hybridization, the hybridisation cocktail was removed and stored at minus 80°C for further use. The arrays were washed and stained in a Affymetrix fluidics station using the phycoerythrin -steptavidin -antibody amplification protocol EukWS2 according to the manufacturers instructions. Data were collected using a scanning confocal microscope made for Affymetrix by Hewlett Packard (Commercially available through Affymetrix, Santa Clara, CA, US.). The scanner uses an argon ion laser as the excitation source, with the emission detected by a photomultiplier tube through either a 530 nm bandpass filter (fluorescein) or a 560 nm longpass filter (phycoerythrin).

### Quantitative analysis of hybridization patterns and intensities:

Following a quantitative scan of an array, a grid is aligned to the image using the known dimensions of the array and the comer control regions as markers. The image is reduced to a simple text file containing position and Intensity Information using software developed at Affymetrix (available with the confocal scanner). This information is merged with another text file that contains information relating physical position on the array to probe sequence and the identify of the RNA (and the specific part of the RNA) for which the oligonucleotide probe Is designed. The quantitative analysis of the hybridization results involves a simple form of pattern recognition based on the assumption that, in the presence of a specific RNA, the PM (PM means "perfect match" in US 6,177,248) probes will hybridize more strongly on average than their MM (MM means "mismatch" in US 6,177,248) partners. The number of instances in which the PM hybridization signal is larger than the MM signal is computed along with average of the logarithm of the PM/MM ratios for each probe set. These values are used to make a decision (using a predefined decision matrix) concerning the presence or absence of an RNA. To determine the quantitative RNA abundance, the average of the differences (PM minus MM) for each probe family is calculated. The advantage of the difference method is that signals from random cross-hybridization contribute equally, on average, to the PM and MM probes, while specific hybridization contributes more to the PM probes. By averaging the pairwise differences, the real signals add constructively while the contributions form cross-hybridization tend to cancel. When assessing the differences between two different RNA samples, the hybridization signals from side-by-side experiments on identically synthesized arrays are compared directly. The magnitude of the changes in the average of the difference (PM-MM) values is interpreted by comparison with the results of spiking experiments as well as the signals observed for the internal standard bacterial and phage RNAs spiked into each sample at a known amount. Data analysis programs developed at Affymetrix perform these operations automatically.

**Table 1**

| | **ZDF rats, 7 weeks old** | | | | |
|---|---|---|---|---|---|
| **Comparison** | ZDF # 1 vs control #1 | ZDF # 2 vs control #2 | ZDF # 3 vs control #3 | ZDF # 4 vs control #4 | ZDF # 5 vs control #5 |
| **Fold change** | - **4.3** | **-4.8** | **-3.1** | **-5.3** | **-4.6** |

**Table 2**

| | **ZDF rats, 9 weeks old** | | | | |
|---|---|---|---|---|---|
| **Comparison** | ZDF 7 vs control #7 | ZDF 8 vs control #8 | ZDF 9 vs control #9 | ZDF 10 vs control #10 | ZDF 11 vs contro 1 #11 |
| **Fold change** | - **5.4** | - **3.3** | - **2.9** | - **1.7** | - **1.3** |

**Table 3**

| | **ZDF rats, 14 weeks old** | | | | |
|---|---|---|---|---|---|
| **Comparison** | ZDF 13 vs control #13 | ZDF 14 vs control #14 | ZDF 15 vs control #15 | ZDF 16 vs control #16 | ZDF 17 vs contro 1 #17 |
| **Fold change** | **1** | - **1.7** | - **3.0** | - **2.4** | - **1.7** |

### Example 4: Gene Expression of PIM-3 in an in vitro model of insulin resistance

Adipocytes were isolated from epidydimal fat pads of 160-180g male Sprague Dawley rats and incubated as described (Müller, G.and Wied, S. Diabetes (1993) 42: 1852-1867). Briefly, adipocytes were isolated from the pooled epidydymal fat of 20 male rats. The isolated adipocytes were split into two pools. One pool was made insulin resistant by incubating the cells for 5 hours in medium containing 25mM D-glucose plus 10nM insulin.

The second pool was incubated for 5h in medium containing 5mM D-glucose, which kept the cells in the insulin sensitive state. At the end of the incubation insulin sensitivity and insulin resistance where checked by measuring insulin dependent glucose uptake with an aliquot of the two pools as described (Müller, G. and Wied, S. Diabetes (1993) 42: 1852-1867). The majority of the adipocytes was used for isolating RNA as described in example 3.

Subsequently RNA was used for gene expression monitoring using Affymetrix technology as described in example 3. Fold changes of gene expression for PIM-3 were analyzed using the Affimetrix qualifier AF086624_S_AT. The results of these analysis are summarized in table 4:

**Table 4**

| | **In vitro insulin resistant adipocytes, 5 h incubated** | | | | |
|---|---|---|---|---|---|
| **Comparison** | Resistant 1 vs control 1 | Resistant 2 vs control 2 | Resistant 3 vs control 3 | Resistant 4 vs control 4 | Resistant 5 vs control 5 |
| **Fold change** | - **2.4** | - **2.7** | - **4.0** | - **2.1** | - **2.4** |

### Experiments 5 : Gene expression of PIM-3 in 3T3-L1 adipocytes treated with antidiabetic drugs (PPARγ agonists)

### Material and Methods:

3T3-L1 preadipocytes were grown at 37°C in 10% CO₂ in Dulbecco's modified Eagle's medium (DMEM) containing 1 g/l glucose and 10% fetal calf serum (FCS). For differentiation into mature adipocytes, confluent preadipocytes were cultured for four days in DMEM supplemented with 4.5 g/l glucose, 10% FCS, 50 µg/ml ascorbic acid, 1 µM biotin, 17 µM pantothenic acid (= basal medium), 500 µM 3-isobutylmethylxanthine, 0.25 µM dexamethasone and 1 µg/ml human recombinant insulin. During the four day treatment the medium was changed once. Finally, 3T3-L1 cells were treated for three days with basal medium containing 1 µg/ml insulin whereupon approximately 90% of the cells were converted into adipocytes.

Differentiated 3T3-L1 cells were maintained in basal medium for one additional day and subsequently kept in serum-free basal medium for four hours. Then, PPARγ agonists diluted in basal medium were added to the adipocytes as described below (concentrations and conditions see table 5):

**Table 5**

| **probe no.** | **compound*** | **concentration** | **duration of treatment** |
|---|---|---|---|
| 1 | rosiglitazone | 1 µM | 6 hours |
| 2 | rosiglitazone | 5 µM | 6 hours |
| 3 | troglitazone | 1 µM | 6 hours |
| 4 | troglitazone | 5 µM | 6 hours |
| 5 | DMSO control | 0.02% (v/v) | 6 hours |
| 6 | rosiglitazone | 1 µM | 24 hours |
| 7 | rosiglitazone | 5 µM | 24 hours |
| 8 | troglitazone | 1 µM | 24 hours |
| 9 | troglitazone | 5 µM | 24 hours |
| 10 | DMSO control. | 0.02% (v/v) | 24 hours |
| 11 | rosiglitazone | 1 µM | 48 hours |
| 12 | rosiglitazone | 5 µM | 48 hours |
| 13 | troglitazone | 1 µM | 48 hours |
| 14 | troglitazone | 5 µM | 48 hours |
| 15 | DMSO control | 0.02% (v/v) | 48 hours |

| | | | |
|---|---|---|---|
| * 5 mM and 25 mM stock solutions of rosiglitazone and troglitazone dissolved in DMSO were made and diluted 5000fold to the final concentrations (1 µM / 5 µM) in the culture medium. For the controls, DMSO without compound was equally diluted 5000fold to a final concentration of 0.02% (v/v). | | | |

RNA was isolated using the TRIzol reagent (Life Technologies, Karlsruhe, Germany) and treated with DNase I by applying the DNA-free kit (Ambion, Austin, TX, US). Further purification of the RNA was achieved using the RNeasy mini kit (Qiagen, Hilden, Germany) and quality/quantity control was done with the 2100Bioanalyzer (Agilent, Böblingen, Germany). 10 µg of total RNA was converted into biotinylated cRNA according to the GeneChip expression analysis technical manual (Affymetrix, Santa Clara, CA, US). Briefly, first and second strand synthesis was performed by applying the Superscript double stranded cDNA synthesis kit (Life Technologies, Karlsruhe, Germany) and biotin labeled cRNA was produced with the BioArray RNA transcript labeling kit (Enzo Diagnostics, Framingdale, NY, US). 10 µg of cRNA was fragmented by heat, added to the GeneChip eukaryotic hybridization control solution (Affymetrix) and hybridized to a GeneChip MG-U74Av2 array (Affymetrix) by rotating for 16 hours at 45°C. Washing, staining and scanning of the array was carried out with standard procedures using the hardware provided by Affymetrix. Raw data was analyzed by applying the microarray suite version 4.0.1 software (Affymetrix, see above).

The entire experiment was repeated twice to provide three biological replicates.

### Data analysis:

Data analysis including the estimation of fold changes was performed as described above. Foldchange values for PIM-3 were obtained by comparing the compound treated samples against the untreated controls for each time point. Therefore, Affymetrix qualifier 96841_AT was used.

The results are summarized in table 6 a) - c).

**Table 6 a)**

| | 6 hours | | | | 24 hours | | | | 48 hours | | | |
|---|---|---|---|---|---|---|---|---|---|---|---|---|
| | Rosi 1µ M | Rosi 5µ M | Tro 1µ M | Tro 5µ M | Rosi 1µ M | Rosi 5µM | Tro 1µM | Tro 5µM | Rosi 1µM | Rosi 5µM | Tro 1µM | Tro 5µM |
| **comparison** | 1/ 5 | 2/ 5 | 3/ 5 | 4/ 5 | 6/ 10 | 7/1 0 | 8/1 0 | 9/1 0 | 11/ 15 | 12/ 15 | 13/ 15 | 14/ 15 |
| **Fold change** | 2. 1 | 2.2 | 1. 7 | 2.1 | 1 9 | 2.9 | 1.8 | 2.7 | 2.6 | 2.6 | 1.8 | 2.4 |

**Table b)**

| | 6 hours | | | | 24 hours | | | | 48 hours | | | |
|---|---|---|---|---|---|---|---|---|---|---|---|---|
| | Rosi 1µ M | Rosi 5µ M | Tro 1µ M | Tro 5µ M | Rosi 1µ M | Rosi 5µM | Tro 1µM | Tro 5µM | Rosi 1µM | Rosi 5µM | Tro 1µM | Tro 5µM |
| **comparison** | 1/ 5 | 2/ 5 | 3/ 5 | 4/ - 5 | 6/ 10 | 7/1 0 | 8/1 0 | 9/1 0 | 11/ 15 | 12/ 15 | 13/ 15 | 14/ 15 |
| **Fold change** | 1. 5 | 1. 3 | 1. 7 | 1. 7 | 2. 2 | 1.9 | 1.6 | 1.9 | 2.0 | 1.9 | 1.6 | 1.9 |

**Table c)**

| | 6 hours | | | | 24 hours | | | | 48 hours | | | |
|---|---|---|---|---|---|---|---|---|---|---|---|---|
| | Rosi 1µ M | Rosi 5µ M | Tro 1µ M | Tro 5µ M | Rosi 1µ M | Rosi 5µM | Tro 1µM | Tro 5µM | Rosi 1µM | Rosi 5µM | Tro 1µM | Tro 5µM |
| **comparison** | 1/ 5 | 2/ 5 | 3/ 5 | 4/ 5 | 6/ 10 | 7/1 0 | 8/1 0 | 9/1 0 | 11/ 15 | 12/ 15 | 13/ 15 | 14/ 15 |
| **Fold change** | 2. 0 | 2. 2 | 1. 8 | 2. 3 | 1. 9 | 1.9 | 1.6 | 2.0 | 2.1 | 2.0 | 1.6 | 2.3 |

| | | | | | | | | | | | | |
|---|---|---|---|---|---|---|---|---|---|---|---|---|
| "Rosi" stands for "rosiglitazone", "Tro" stands for "troglitazone". | | | | | | | | | | | | |

### Example 6 : Gene expression of Pim-3 in ZDF rats treated with anti-diabetic drugs (PPARγ agonists)

Zucker Diabetic Fatty (ZDF/Gmi.TM.-fa/fa) male rats as well as their lean fa+/fa-counterparts, aged 5 weeks (control 1-5, ZDF 1-10)were obtained from Genetic Models Inc. (Indianapolis, Ind.). The rats were given free access to food and water. The ZDF animals were splitted into 2 groups: group 1 (animals ZDF 1-5) was not treated with any agent, whereas the second group was treated for 14 weeks with the antidiabetic drug Rosiglitazone (3 mg/kg/day, animals 6-10, Rosi 1-5,). For the collection of epididymal fat pads and also blood samples, in each case 5 animals were sacrificed by cervical dislocation. To monitor the success of the antidiabetic treatment, HbA1c as marker of long-term blood glucose levels were measured by standard methods after finishing of the experiment. The results of these measurements are summarized in table 7:

**Table 7:**

| | % HbA1c in different rats: controls rats (C), ZDF rats untreated (Z), ZDF rats Rosiglitazone treated (R) | | | | | | | | | | | | | | |
|---|---|---|---|---|---|---|---|---|---|---|---|---|---|---|---|
| animal | C 1 | C2 | C3 | C4 | C5 | Z1 | Z2 | Z3 | Z4 | Z5 | R1 | R2 | R3 | R4 | R5 |
| HbA1c (%) | 4.20 | 4.28 | 4.21 | 4.16 | 4.09 | 5.12 | 8.01 | 7.59 | 8.71 | 9.51 | 4.30 | 4.27 | 4.14 | 4.14 | 4.13 |

Collection of epididymal adipose tissue, RNA isolation and Affymetrix experiments were performed as described in experiment 1. Data analysis included the comparison of expression data of samples derived from the lean control animals versus the untreated ZDF rats as well as the comparison of the expression data derived from Rosiglitazone treated ZDF animals versus non-treated ZDF animals. Data analysis including the estimation of fold changes and weighing of the statistical significance of those fold changes was performed using a proprietary software developed in Aventis pharmaceuticals. Fold changes of gene expression for Pim-3 were analyzed using the Affimetrix qualifier AF086624_S_AT. The results of those analysis are summarised in the following tables 8a und 8b:

**Table 8a:**

| | **ZDF rats, 20 weeks old** | | | | |
|---|---|---|---|---|---|
| **compari son** | ZDF 1 vs control #1 | ZDF 2 vs control #2 | ZDF 3 vs control #3 | ZDF 4 vs control #4 | ZDF 5 vs control #5 |
| **Fold change** | **-1.5** | **-1.8** | **-1.6** | **1** | **-1.7** |

**Table 8b:**

| | **ZDF rats Rosiglitazone treated, comparison to untreated ZDF rats, 20 weeks old** | | | | |
|---|---|---|---|---|---|
| **compari son** | Rosi 1 vs ZDF 1 | Rosi 2 vs ZDF 2 | Rosi 3 vs ZDF 3 | Rosi 4 vs ZDF 4 | Rosi 5 vs ZDF 5 |
| **Fold change** | **1.6** | **1.3** | **1.6** | **1.5** | **1.4** |

### # SEQUENCE LISTING

<160> NUMBER OF SEQ ID NOS: 14
<210> SEQ ID NO 1
<211> LENGTH: 1973
<212> TYPE: DNA
<213> ORGANISM: Homo sapiens
- - <400> SEQUENCE: 1 
1 GAGGGCCGTC GCCCGCGATG CTGCTCTCCA AGTTCGGCTC CCTGGCGCAC
51 CTCTGCGGGC CCGGCGGCGT GGACCACCTC CCGGTGAAGA TCCTGCAGCC
101 AGCCAAGGCG GACAAGGAGA GCTTCGAGAA GGCGTACCAG GTGGGCGCCG
151 TGCTGGGTAG CGGCGGCTTC GGCACGGTCT ACGCGGGTAG CCGCATCGCC
201 GACGGGCTCC CGGTGGCTGT GAAGCACGTG GTGAAGGAGC GGGTGACCGA
251 GTGGGGCAGC CTGGGCGGCG CGACCGTGCC CCTGGAGGTG GTGCTGCTGC
301 GCAAGGTGGG CGCGGCGGGC GGCGCGCGCG GCGTCATCCG CCTGCTGGAC
351 TGGTTCGAGC GGCCCGACGG CTTCCTGCTG GTGCTGGAGC GGCCCGAGCC
401 GGCGCAGGAC CTCTTCGACT TTATCACGGA GCGCGGCGCC CTGGACGAGC
451 CGCTGGCGCG CCGCTTCTTC GCGCAGGTGC TGGCCGCCGT GCGCCACTGC
501 CACAGCTGCG GGGTCGTGCA CCGCGACATT AAGGACGAAA ATCTGCTTGT
551 GGACCTGCGC TCCGGAGAGC TCAAGCTCAT CGACTTCGGT TCGGGTGCGC
601 TGCTCAAGGA CACGGTCTAC ACCGACTTCG ACGGCACCCG AGTGTACAGC
651 CCCCCGGAGT GGATCCGCTA CCACCGCTAC CACGGGCGCT CGGCCACCGT
701 GTGGTCGCTG GGCGTGCTTC TCTACGATAT GGTGTGTGGG GACATCCCCT
751 TCGAGCAGGA CGAGGAGATC CTCCGAGGCC GCCTGCTCTT CCGGAGGAGG
801 GTCTCTCCAG AGTGCCAGCA GCTGATCCGG TGGTGCCTGT CCCTGCGGCC
851 CTCAGAGCGG CCGTCGCTGG ATCAGATTGC GGCCCATCCC TGGATGCTGG
901 GGGCTGACGG GGGCGCCCCG GAGAGCTGTG ACCTGCGGCT GTGCACCCTC
951 GACCCTGATG ACGTGGCCAG CACCACGTCC AGCAGCGAGA GCTTGTGAGG
1001 AGCTGCACCT GACTGGGAGC TAGGGGACCA CCTGCCTTGG CCAGACCTGG
1051 GACGCCCCCA GACCCTGACT TTTTCCTGCG TGGGCCGTCT CCTCCTGCGG
1101 AAGCAGTGAC CTCTGACCCC TGGTGACCTT CGCTTTGAGT GCCTTTTGAA
 
1151 CGCTGGTCCC GCGGGACTTG GTTTTCTCAA GCTCTGTCTG TCCAAAGACG
1201 CTCCGGTCGA GGTCCCGCCT GCCCTGGGTG GATACTTGAA CCCCAGACGC
1251 CCCTCTGTGC TGCTGTGTCC GGAGGCGGCC TTCCCATCTG CCTGCCCACC
1301 CGGAGCTCTT TCCGCCGGCG CAGGGTCCCA AGCCCACCTC CCGCCCTCAG
1351 TCCTGCGGTG TGCGTCTGGG CACGTCCTGC ACACACAATG CAAGTCCTGG
1401 CCTCCGCGCC CGCCCGCCCA CGCGAGCCGT ACCCGCCGCC AACTCTGTTA
1451 TTTATGGTGT GACCCCCTGG AGGTGCCCTC GGCCCACCGG GGCTATTTAT
1501 TGTTTAATTT ATTTGTTGAG GTTATTTCCT CTGAGCAGTC TGCCTCTCCC
1551 AAGCCCCAGG GGACAGTGGG GAGGCAGGGG AGGGGGTGGC TGTGGTCCAG
1601 GGACCCCAGG CCCTGATTCC TGTGCCTGGC GTCTGTCCTG GCCCCGCCTG
1651 TCAGAAGATG AACATGTATA GTGGCTAACT TAAGGGGAGT GGGTGACCCT
1701 GACACTTCCA GGCACTGTGC CCAGGGTTTG GGTTTTAAAT TATTGACTTT
1751 GTACAGTCTG CTTGTGGGCT CTGAAAGCTG GGGTGGGGCC AGAGCCTGAG
1801 CGTTTAATTT ATTCAGTACC TGTGTTTGTG TGAATGCGGT GTGTGCAGGC
1851 ATCGCAGATG GGGGTTCTTT CAGTTCAAAA GTGAGATGTC TGGAGATCAT
1901 ATTTTTTTAT ACAGGTATTT CAATTAAAAT GTTTTTGTAC ATAGTGGAAA
1950 AAAAAAAAAA AAGGGCGGCC GCC
<210> SEQ ID NO 2
<211> LENGTH: 326
<212> TYPE: PRT
<213> ORGANISM: Homo sapiens
<400> SEQUENCE: 2 
1 MLLSKFGSLA HLCGPGGVDH LPVKILQPAK ADKESFEKAY QVGAVLGSGG
51 FGTVYAGSRI ADGLPVAVKH VVKERVTEWG SLGGATVPLE VVLLRKVGAA
101 GGARGVIRLL DWFERPDGFL LVLERPEPAQ DLFDFITERG ALDEPLARRF
151 FAQVLAAVRH CHSCGVVHRD IKDENLLVDL RSGELKLIDF GSGALLKDTV
201 YTDFDGTRVY SPPEWIRYHR YHGRSATVWS LGVLLYDMVC GDIPFEQDEE
251 ILRGRLLFRR RVSPECQQLI RWCLSLRPSE RPSLDQIAAH PWMLGADGGA
301 PESCDLRLCT LDPDDVASTT SSSESL
 <210> SEQ ID NO 3.
<211> LENGTH: 978
<212> TYPE: DNA
<213> ORGANISM: Homo sapiens
<400> SEQUENCE: 3 
1 ATGCTGCTCT CCAAGTTCGG CTCCCTGGCG CACCTCTGCG GGCCCGGCGG
51 CGTGGACCAC CTCCCGGTGA AGATCCTGCA GCCAGCCAAG GCGGACAAGG
101 AGAGCTTCGA GAAGGCGTAC CAGGTGGGCG CCGTGCTGGG TAGCGGCGGC
151 TTCGGCACGG TCTACGCGGG TAGCCGCATC GCCGACGGGC TCCCGGTGGC
201 TGTGAAGCAC GTGGTGAAGG AGCGGGTGAC CGAGTGGGGC AGCCTGGGCG
251 GCGCGACCGT GCCCCTGGAG GTGGTGCTGC TGCGCAAGGT GGGCGCGGCG
301 GGCGGCGCGC GCGGCGTCAT CCGCCTGCTG GACTGGTTCG AGCGGCCCGA
351 CGGCTTCCTG CTGGTGCTGG AGCGGCCCGA GCCGGCGCAG GACCTCTTCG
401 ACTTTATCAC GGAGCGCGGC GCCCTGGACG AGCCGCTGGC GCGCCGCTTC
451 TTCGCGCAGG TGCTGGCCGC CGTGCGCCAC TGCCACAGCT GCGGGGTCGT
501 GCACCGCGAC ATTAAGGACG AAAATCTGCT TGTGGACCTG CGCTCCGGAG
551 AGCTCAAGCT CATCGACTTC GGTTCGGGTG CGCTGCTCAA GGACACGGTC
601 TACACCGACT TCGACGGCAC CCGAGTGTAC AGCCCCCCGG AGTGGATCCG
651 CTACCACCGC TACCACGGGC GCTCGGCCAC CGTGTGGTCG CTGGGCGTGC
701 TTCTCTACGA TATGGTGTGT GGGGACATCC CCTTCGAGCA GGACGAGGAG
751 ATCCTCCGAG GCCGCCTGCT CTTCCGGAGG AGGGTCTCTC CAGAGTGCCA
801 GCAGCTGATC CGGTGGTGCC TGTCCCTGCG GCCCTCAGAG CGGCCGTCGC
851 TGGATCAGAT TGCGGCCCAT CCCTGGATGC TGGGGGCTGA CGGGGGCGCC
901 CCGGAGAGCT GTGACCTGCG GCTGTGCACC CTCGACCCTG ATGACGTGGC
951 CAGCACCACG TCCAGCAGCG AGAGCTTG
<211> LENGTH: 254
<212> TYPE: PRT
<213> ORGANISM: Artificial Sequence
<220> FEATURE:
<223> OTHER INFORMATION: Description of Artificial Sequence: eukaryotic
   protein kinase domain
<400> SEQUENCE: 4 
1 YQVGAVLGSG GFGTVYAGSRI ADGLPVAVKH VVKERVTEWG SLGGATVPL
51 EVVLLRKVGA AGGARGVIRLL DWFERPDGFL LVLERPEPAQ DLFDFITER
101 GALDEPLARR FFAQVLAAVRH CHSCGVVHRD IKDENLLVDL RSGELKLID
151 FGSGALLKDT VYTDFDGTRVY SPPEWIRYHR YHGRSATVWS LGVLLYDMV
201 CGDIPFEQDE EILRGRLLFRR RVSPECQQLI RWCLSLRPSE RPSLDQIAA
251 HPWM
<210> SEQ ID NO:5
<211> LENGTH: 2236
<212> TYPE: DNA
<213> ORGANISM: Mus musculus
<400> SEQUENCE: 5 
1 TGACACTATA GAAGGTACGC CTGCAGGTAC CGGTCCGGAA TTCCCGGGTC
51 GACCCACGCG TCCGCGCCGC GCGGCTGCCC CGCTGAGCGC TCGGCCTCGG
101 GGCCGTGGGA TCCGCCGCGC TGTCTGCGGT CAGGAAGACC GCCCTCCCGC
151 GTCCTTGCCG GACGGGTCAG AGGCGGCACC GCACGCGAGG CCACCCGCGA
201 TGCTGCTGTC CAAGTTCGGC TCCCTGGCGC ACCTCTGCGG GCCTGGCGGC
251 GTGGACCACC TCCCAGTGAA GATCCTACAG CCAGCCAAGG CTGACAAGGA
301 GAGCTTCGAG AAGGTGTACC AGGTGGCGCC CGTGCTGGGC AGCGGCGGCT
351 TCGGCACGGT CTACGCGGGC AGCCGCATCG CCGACGGACT CCCGGTGGCT
401 GTGAAGCACG TGGTGAAGGA GCGGGTGACC GAGTGGGGCA GTCTCGGCGG
451 AGTGGCCGTG CCCCTGGAGG TGGTGCTGCT GCGCAAGGTG GGCGCGGCGG
501 GCGGCGCGCG CGGCGTCATC CGCTTGCTGG ACTGGTTCGA GCGGCCCGAC
551 GGCTTCTTGT TGGTGCTGGA GCGACCCGAG CCGGCACAGG ACCTCTTCGA
 
601 CTTCATCACT GAACGAGGCG CCCTGGACGA GCCGCTGGCG CGTCGCTTCT
651 TCGCGCAGGT GCTTGCCGCT GTGCGGCACT GCCACAATTG TGGGGTCGTG
701 CACCGCGACA TCAAGGACGA GAACCTGCTG GTGGACCTGC GCTCGGGAGA
751 GCTGAAGCTC ATCGACTTCG GCTCGGGCGC GGTGCTCAAG GACACGGTCT
801 ACACTGACTT TGATGGCACC CGTGTGTACA GCCCCCCAGA GTGGATCCGA
851 TATCACCGAT ATCACGGGCG GTCTGCCACT GTGTGGTCTC TGGGTGTACT
901 GCTCTACGAC ATGGTGTGTG GGGACATTCC CTTTGAGCAG GATGAGGAGA
951 TCTTGCGCGG CAGGCTCTTT TTCCGGAGGA GGGTCTCCCC AGAGTGCCAG
1001 CAGCTTATTG AGTGGTGTCT CTCCCTGAGG CCCTCAGAGA GGCCCTCCCT
1051 GGACCAAATT GCTGCCCACC CCTGGATGCT GGGGACAGAG GGGAGCGTTC
1101 CAGAGAACTG TGACCTTCGG CTTTGTGCCC TGGATACTGA CGACGGAGCC
1151 AGTACCACTT CCAGCAGTGA GAGCTTGTGA GGAGGAGAAG GGGCCTGGGC
1201 TCGGCCTAGC CAGCGCTCTC CCAGAATTGA ACACTTTCTG CCTGGGATGT
1251 CTGCTGCAAA AGCAGTGACC TCTGACCCCT GGTGACCTTT GCTCTCGGCA
1301 CCGGGCCTGT TTCCTTTGCT TTGAGTGCCT TTTTGAACGC TGCTCCACAG
1351 GGCCTGGGTT TTCTTGAGCT CTTCTGTCCA AAGATGGCTG CGGGCTAAGC
1401 AAGGTCCTGC CCTGGGTGGA TACTTGAACC AGAGATCCCG ACCCTGCTGC
1451 TCCATCTCAG GAGGCAGCCT TCCTGACCAA GTGTGTTTGA CATGGAGCGC
1501 CCTGTGGTGC CCACCTCCAA CCCTCCAGTC TCCTGGTGTT CATCTGGGCA
1551 TGTCTGCACA AGCAATGCAA CGCTGGGCCA CTGCTGCCCG TCTGCCTCCC
1601 CGGCACGGCA CGGCTCCGCA CGCAACCTAA GCGTGCCACC ACGGTCTCTT
1651 ATTTATGGTG TGATCACCCT GGAGGGCGCC CCCGCCCTGC TGGGGCTATT
1701 TATTGTTTAA TTTATTTGCT GAGGTTCCTC CAAGCAACCA CCTTCTCCAG
1751 GCCCCTGGGG TGTTGAAAGT CAAATGTGGC TGTTGAGTCC ACAGACCCCC
1801 ATCCTAATTC CTGCACCTGG AGGAGTTCCC CAACCCCCGT GTTTGCGGGA
1851 GGAAGCATTT GTACAGTGGC TAATTTAAGG GGAGTGGGAG ACCCTGTCAC
1901 CCTGAGCACT CTGCGCTGGG GAGGGGTTTA AATTATTGAC CTTGTACAGT
1951 CTGCTTGCTG GCTCTGAAAG CTGGGGTTGG GGGACAGAGT CTCAAGCCCT
2001 TAATTTATTT TAGCAGCTGT GTTTCTGTGA CCCTGGTGTG ACTAAGCATC
2051 AGGGGTGGGG TTGTATAAGT TCAAAAGTGT GAAATGTCTG AAGATCATAT
2101 TTTTTATACA GGTATTTCAA TTAAATGTTT TGGTATATAA AAAAAAAAAA
2151 AAAAAAAAAA AAGGGCGGCC GCTCTAGAGG ATCCCTCGAG GGGCCCAAGC
2201 TTACGCGTGC ATGCGACGTC ATAGCTCTCT CCCTAT
<210> SEQ ID NO:6
<211> LENGTH: 326
<212> TYPE: PRT <213> ORGANISM: Mus musculus
<400> SEQUENCE: 6 
1 MLLSKFGSLA HLCGPGGVDH LPVKILQPAK ADKESFEKVY QVGAVLGSGG
51 FGTVYAGSRI ADGLPVAVKH VVKERVTEWG SLGGVAVPLE VVLLRKVGAA
101 GGARGVIRLL DWFERPDGFL LVLERPEPAQ DLFDFITERG ALDEPLARRF
151 FAQVLAAVRH CHNCGVVHRD IKDENLLVDL RSGELKLIDF GSGAVLKDTV
201 YTDFDGTRVY SPPEWIRYHR YHGRSATVWS LGVLLYDMVC GDIPFEQDEE
251 ILRGRLFFRR RVSPECQQLI EWCLSLRPSE RPSLDQIAAH PWMLGTEGSV
301 PENCDLRLCA LDTDDGASTT SSSESL
<210> SEQ ID NO:7
<211> LENGTH: 978
<212> TYPE: DNA
<213> ORGANISM: Mus musculus
<400> SEQUENCE: 7 
1 ATGCTGCTGT CCAAGTTCGG CTCCCTGGCG CACCTCTGCG GGCCTGGCGG
51 CGTGGACCAC CTCCCAGTGA AGATCCTACA GCCAGCCAAG GCTGACAAGG
101 AGAGCTTCGA GAAGGTGTAC CAGGTGGGCG CCGTGCTGGG CAGCGGCGGC
151 TTCGGCACGG TCTACGCGGG CAGCCGCATC GCCGACGGAC TCCCGGTGGC
201 TGTGAAGCAC GTGGTGAAGG AGCGGGTGAC CGAGTGGGGC AGTCTCGGCG
251 GAGTGGCCGT GCCCCTGGAG GTGGTGCTGC TGCGCAAGGT GGGCGCGGCG
301 GGCGGCGCGC GCGGCGTCAT CCGCTTGCTG GACTGGTTCG AGCGGCCCGA
351 CGGCTTCTTG TTGGTGCTGG AGCGACCCGA GCCGGCACAG GACCTCTTCG
401 ACTTCATCAC TGAACGAGGC GCCCTGGACG AGCCGCTGGC GCGTCGCTTC
451 TTCGCGCAGG TGCTTGCCGC TGTGCGGCAC TGCCACAATT GTGGGGTCGT
 
501 GCACCGCGAC ATCAAGGACG AGAACCTGCT GGTGGACCTG CGCTCGGGAG
551 AGCTGAAGCT CATCGACTTC GGCTCGGGCG CGGTGCTCAA GGACACGGTC
601 TACACTGACT TTGATGGCAC CCGTGTGTAC AGCCCCCCAG AGTGGATCCG
651 ATATCACCGA TATCACGGGC GGTCTGCCAC TGTGTGGTCT CTGGGTGTAC
701 TGCTCTACGA CATGGTGTGT GGGGACATTC CCTTTGAGCA GGATGAGGAG
751 ATCTTGCGCG GCAGGCTCTT TTTCCGGAGG AGGGTCTCCC CAGAGTGCCA
801 GCAGCTTATT GAGTGGTGTC TCTCCCTGAG GCCCTCAGAG AGGCCCTCCC
851 TGGACCAAAT TGCTGCCCAC CCCTGGATGC TGGGGACAGA GGGGAGCGTT
901 CCAGAGAACT GTGACCTTCG GCTTTGTGCC CTGGATACTG ACGACGGAGC
951 CAGTACCACT TCCAGCAGTG AGAGCTTG
 <211> LENGTH: 254
<212> TYPE: PRT
<213> ORGANISM: Artificial Sequence
<220> FEATURE:
<223> OTHER INFORMATION: Description of Artificial Sequence: eukaryotic
   protein kinase domain
<400> SEQUENCE: 8 
1 YQVGAVLGSG GFGTVYAGSR IADGLPVAVK HVVKERVTEW GSLGGVAVPL
51 EVVLLRKVGA AGGARGVIRL LDWFERPDGF LLVLERPEPA QDLFDFITER
101 GALDEPLARR FFAQVLAAVR HCHNCGVVHR DIKDENLLVD LRSGELKLID
151 FGSGAVLKDT VYTDFDGTRV YSPPEWIRYH RYHGRSATVW SLGVLLYDMV
201 CGDIPFEQDE EILRGRLFFR RRVSPECQQL IEWCLSLRPS ERPSLDQIAA
251 HPWM
<210> SEQ ID NO:9
<211> LENGTH: 2133
<212> TYPE: DNA
<213> ORGANISM: Rattus norvegicus
<400> SEQUENCE: 9 
1 CGCTCGGCCA GCTGCCGTCT ACGGGCTTCC GCGCGGCCAC CGGGCAACTG
51 CGCCGCGCGG CTGCCCCACT GAGCGCTCGG CCTCGGGGCC GTGGGATCCG
101 CCGCGCTGTC TGCGGTCAGG AAGACCGCCC TCCCGCGTCC GTGCCGGACG
151 GGTCAGAGGC GGCGCCGCAC GCGAGGCCAC CCGCGATGCT GCTGTCCAAG
201 TTCGGCTCCC TGGCGCACCT CTGCGGGCCT GGCGGCGTGG ACCACCTCCC
251 AGTGAAGATC CTACAGCCAG CCAAGGCGGA CAAGGAGAGC TTCGAGAAGG
301 TGTACCAGGT GGGCGCCGTG CTCGGCAGCG GCGGCTTCGG CACGGTCTAC
351 GCGGGCAGCC GCATCGCCGA CGGACTCCCG GTGGCTGTGA AGCACGTGGT
401 GAAGGAGCGG GTGACCGAGT GGGGCAGTCT CGGCGGAATG GCCGTGCCCC
451 TGGAGGTGGT GCTGCTGCGC AAGGTGGGCG CGGCGGGCGG CGCGCGCGGC
501 GTCATCCGCC TGCTGGACTG GTTCGAGCGG CCCGACGGCT TCCTGCTGGT
551 GCTGGAGCGA CCCGAGCCGG CACAGGACCT CTTCGACTTC ATCACTGAAC
601 GCGGCGCCCT GGACGAGCCT CTGGCTCGTC GCTTCTTCGC GCAGGTGCTC
651 GCCGCTGTGC GGCACTGCCA CAATTGTGGG GTCGTGCACC GCGACATCAA
701 GGACGAGAAC CTGCTGGTGG ACTTGCGCTC GGGCGAGCTG AAGCTCATCG
751 ACTTCGGCTC GGGCGCGGTG CTCAAGGACA CGGTCTACAC TGACTTTGAT
801 GGCACCCGTG TGTACAGCCC CCCAGAGTGG ATCCGGTATC ATCGATATCA
851 CGGGCGGTCT GCCACTGTGT GGTCTCTGGG TGTACTGCTC TACGACATGG
901 TGTGTGGGGA CATTCCCTTT GAGCAGGATG AGGAGATCTT GCGCGGCAGG
951 CTCTTTTTCC GGAGGAGGGT CTCCCCAGAG TGCCAGCAGC TTATTGAGTG
1001 GTGTCTCTCC CTGCGGCCCT CAGAGAGGCC CTCGCTGGAC CAAATTGCTG
1051 CCCATCCCTG GATGCTGGGG ACAGAGGGCA GCGTTCCAGA GAACTGTGAC
1101 CTTCGGCTCT GTGCCCTGGA TACTGATGAC GGAGCCAGTA CCACTTCCAG
1151 CAGTGAGAGC TTGTGAGGAG GAGGAGGGGC CTGGACTCCA CACTGGGGGC
1201 CTGGGCTCAG CCTAGCCAGC CCTCTCCCAG AATGAACATT TTCTGCCTGG
1251 GATGTCTCCT GCAAAAGCAG TGACCTCTGA CCCCTGGTGA CCTTTGCTCT
1301 CGGCACCGGG CCTGTTTCCT TTGCTTTGAG TGCCTTTTTG AACGCTGCTC
 
1351 CACAGGGCCT GGGTTTTCTT GAGCTCTTCT GTCCAAAGAT GGCTGCGGGC
1401 TAAGCAAGGT CCCGCCTGCC CTGGGTGGAT ACTTGAACCC GAGACCCTAC
1451 CCTGCTGCTC CATCTTGCGG CAGCCTTCCT GACCAAGTGT GTTTGACATG
1501 GAGCGCCCTG TGGTGCCCAC CTCCAACCCT CCAGTCTCCT GGTCTTCGTC
1551 TGGGCATGTC TGCACAAGCA ATGCAACGCT GGGCCACTGC TGCCCGCCTG
1601 CCTCCCTGGC ACCGCACGCA ACGAGCGTGC CACGGTCTCT TATTTATGGT
1651 GTGATCACCC TGGAGGGCGC CCCTGCCCTG CTGGGCTAT TTATTGTTTA
1701 ATTTATTTGC TGAGGTTACT TCCTCCAAGC AACCACCTTC TCCAGGCCCC
1751 TGGGGTGTTC AGGAAAGCCA AGGGTGGCCG TTCAGTCCAC AGACGGCATC
1801 CTGGTTCCTG CACCTGCAGT AGGTCCCTAA CCCCATGTTT GTGGAGGAG
1851 GAATTTGTAC AGTGGCTAAT TTAAGGGGAG TGGGAGACCC TGTCACCCTG
1901 GGCACTCTGC GCTGGGGAGG GGGTTTAAAT TATTGACCTT GTACAGTCTG
1951 CTTGCTGGCT CTGAAAGCTG GGGTGGGGGA CAGAGTCTCA AGCCCTTAAT
2001 TTATTTTAGC AACTGTGTTC TGTGACCCTG GTGTGAGTAG GCATCAGGGG
2051 TGGGGTTGTA TAAGTTCAAA AGTGTGAAAT GTCTGGAGAT CATATTTTTT
2101 ATACAGGTAT TTCAATTAAA TGTTTTGGTA TAT
<210> SEQ ID NO:10
<211> LENGTH:326
<212> TYPE: PRT
<213> ORGANISM: Rattus norvegicus
<400> SEQUENCE:10 
1 MLLSKFGSLA HLCGPGGVDH LPVKILQPAK ADKESFEKVY QVGAVLGSGG
51 FGTVYAGSRI ADGLPVAVKH VVKERVTEWG SLGGMAVPLE VVLLRKVGAA
101 GGARGVIRLL DWFERPDGFL LVLERPEPAQ DLFDFITERG ALDEPLARRF
151 FAQVLAAVRH CHNCGVVHRD IKDENLLVDL RSGELKLIDF GSGAVLKDTV
201 YTDFDGTRVY SPPEWIRYHR YHGRSATVWS LGVLLYDMVC GDIPFEQDEE
251 ILRGRLFFRR RVSPECQQLI EWCLSLRPSE RPSLDQIAAH PWMLGTEGSV
301 PENCDLRLCA LDTDDGASTT SSSESL
<210> SEQ ID NO:11
<211> LENGTH: 323
<212> TYPE: PRT
<213> ORGANISM: Xenopus laevis
<400> SEQUENCE:11 
1 MLLSKFGSLA HICNPSNMEH LPVKILQPVK VDKEPFEKVY QVGSVVASGG
51 FGTVYSDSRI ADGQPVAVKH VAKERVTEWG TLNGVMVPLE IVLLKKVPTA
101 FRGVINLLDW YERPDAFLIV MERPEPVKDL FDYITEKGPL DEDTARGFFR
151 QVLEAVRHCY NCGVVHRDIK DENLLVDTRN GELKLIDFGS GALLKDTVYT
201 DFDGTRVYSP PEWVRYHRYH GRSATVWSLG VLLYDMVYGD IPFEQDEEIV
251 RVRLCFRRRI STECQQLIKW CLSLRPSDRP TLEQIFDHPW MCKCDLVKSE
301 DCDLRLRTID NDSSSTSSSN ESL
<210> SEQ ID NO:12
<211> LENGTH: 313
<212> TYPE: PRT
<213> ORGANISM: Rattus norvegicus
<400> SEQUENCE:12 
1 MLLSKINSLA HLRAAPCNDL HANKLAPGKE KEPLESQYQV GPLLGSGGFG
51 SVYSGIRVAD NLPVAIKHVE KDRISDWGEL PNGTRVPMEV VLLKKVSSGF
101 SGVIRLLDWF ERPDSFVLIL ERPEPVQDLF DFITERGALQ EELARSFFWQ
151 VLEAVRHCHN CGVLHRDIKD ENILIDLNRG ELKLIDFGSG ALLKDTVYTD
201 FDGTRVYSPP EWIRYHRYHG RSAAVWSLGI LLYDMVCGDI PFEHDEEIVK
251 GQVYFRQRVS SECQHLIRWC LSLRPSDRPS FEEIQNHPWM QDVLLPQATA
301 EIHLHSLSPS PSK
<210> SEQ ID NO:13
<211> LENGTH: 313
<212> TYPE: PRT
<213> ORGANISM: Homo sapiens
<400> SEQUENCE:13 
1 MLLSKINSLA HLRAAPCNDL HATKLAPGKE KEPLESQYQV GPLLGSGGFG
51 SVYSGIRVSD NLPVAIKHVE KDRISDWGEL PNGTRVPMEV VLLKKVSSGF
101 SGVIRLLDWF ERPDSFVLIL ERPEPVQDLF DFITERGALQ EELARSFFWQ
151 VLEAVRHCHN CGVLHRDIKD ENILIDLNRG ELKLIDFGSG ALLKDTVYTD
201 FDGTRVYSPP EWIRYHRYHG RSAAVWSLGI LLYDMVCGDI PFEHDEEIIR
251 GQVFFRQRVS SECQHLIRWC LALRPSDRPT FEEIQNHPWM QDVLLPQETA
301 EIHLHSLSPG PSK
<210> SEQ ID NO:14
<211> LENGTH: 313
<212> TYPE: PRT
<213> ORGANISM: Mus musculus
<400> SEQUENCE:14

1 MLLSKINSLA HLRARPCNDL HATKLAPGKE KEPLESQYQV GPLLGSGGFG
51 SVYSGIRVAD NLPVAIKHVE KDRISDWGEL PNGTRVPMEV VLLKKVSSDF
101 SGVIRLLDWF ERPDSFVLIL ERPEPVQDLF DFITERGALQ EDLARGFFWQ
151 VLEAVRHCHN CGVLHRDIKD ENILIDLSRG EIKLIDFGSG ALLKDTVYTD
201 FDGTRVYSPP EWIRYHRYHG RSAAVWSLGI LLYDMVCGDI PFEHDEEIIK
251 GQVFFRQTVS SECQHLIKWC LSLRPSDRPS FEEIRNHPWM QGDLLPQAAS
301 EIHLHSLSPG SSK

## Claims

1. Use of a PIM-3 polypeptide as a screening agent for identifying anti-type 2 diabetes mellitus drugs, said polypeptide being selected from a polypeptide having amino acid sequence SEQ ID NO. 2, 6 or 10.

2. Use of PIM-3 encoding DNA as a screening agent for identifying anti-type 2 diabetes mellitus drugs, said DNA comprises nucleotide sequence SEQ ID NO. 1, 5 or 9.

3. PIM-3 polypeptide for use in the treatment of insulin resistance or type 2 diabetes mellitus said polypeptide having amino acid sequence SEQ ID NO. 2, 6, or 10.

## Patentansprüche

1. Verwendung eines PIM-3-Polypeptids als Screening-Mittel zur Identifizierung von Arzneimitteln gegen Typ-2-Diabetes mellitus, wobei das Polypeptid aus Polypeptiden mit der Aminosäuresequenz SEQ ID Nr. 2, 6 oder 10 ausgewählt ist.

2. Verwendung einer für PIM-3 codierenden DNA als Screening-Mittel zum Identifizieren von Arzneimitteln gegen Typ-2-Diabetes mellitus, wobei die DNA die Nukleotidsequenz SEQ ID Nr. 1, 5 oder 9 umfasst.

3. PIM-3-Polypeptide zur Verwendung bei der Behandlung von Insulinresistenz oder Typ-2-Diabetes mellitus, wobei die Polypeptide die Aminosäuresequenz SEQ ID Nr. 2, 6 oder 10 aufweisen.

## Revendications

1. Utilisation d'un polypeptide de PIM-3 en tant qu'agent de dépistage pour l'identification de médicaments contre le diabète de type 2, ledit polypeptide étant choisi parmi un polypeptide ayant une séquence d'acides aminés SEQ ID NO. 2, 6 ou 10.

2. Utilisation d'ADN codant pour PIM-3 en tant qu'agent de dépistage pour l'identification de médicaments contre le diabète de type 2, ledit ADN comprenant la séquence nucléotidique SEQ ID NO. 1, 5 ou 9.

3. Polypeptide de PIM-3, destiné à être utilisé dans le traitement de la résistance à l'insuline ou du diabète de type 2, ledit polypeptide ayant la séquence d'acides aminés SEQ ID NO. 2, 6 ou 10.
